# FASCICULE DE BREVET EUROPEEN

(11) **EP 2 373 818 B1**
(45) Date de publication et mention de la délivrance du brevet: **30.03.2016**
(21) Numéro de dépôt: 10706692.0
(22) Date de dépôt: 04.01.2010
(51) Int. Cl.: C12Q 1/68

(54) **PROCEDE D'AMPLIFICATION ET/OU DE DETECTION D'ACIDES NUCLEIQUES ET UTILISATIONS DE CE PROCEDE**
VERFAHREN ZUR VERSTÄRKUNG UND/ODER ERKENNUNG VON NUKLEINSÄUREN UND VERWENDUNGEN DES VERFAHRENS
METHOD FOR AMPLIFYING AND/OR DETECTING NUCLEIC ACIDS AND USES OF SAID METHOD

(30) Priorité: 05.01.2009 FR 0950013
(43) Date de publication de la demande: 12.10.2011
(73) Titulaire: Biomérieux, 69280 Marcy L'etoile (FR)
(72) Inventeur: LAAYOUN, Ali, F-38260 La Frette (FR); TROESCH, Alain, F-69740 Genas (FR)
(86) Numéro de dépôt international: PCT/FR2010/050001
(87) Numéro de publication internationale: WO 2010/076546

(56) Documents cités:
- WO-A-2006/040187
- WO-A-2006/058393
- US-B1- 6 248 522
- VEEDU RAKESH N ET AL: "Novel applications of locked nucleic acids." NUCLEIC ACIDS SYMPOSIUM SERIES (2004) 2007, no. 51, 2007, pages 29-30, XP002543301 ISSN: 1746-8272
- T. Mohammadi ET AL: "Optimization of Real-Time PCR Assay for Rapid and Sensitive Detection of Eubacterial 16S Ribosomal DNA in Platelet Concentrates", Journal of clinical microbiology, vol. 41, no. 10, 1 October 2003 (2003-10-01), pages 4796-4798, XP055167906, ISSN: 0095-1137, DOI: 10.1128/JCM.41.10.4796-4798.2003

## Description

La présente invention concerne un procédé d'amplification permettant de s'affranchir de contaminants dans un échantillon biologique liquide contenant des acides nucléiques d'intérêt que l'on souhaite amplifier, le procédé dans lequel on traite enzymatiquement l'échantillon biologique pour permettre la conversion d'une des bases desdits acides nucléiques d'intérêt en une autre base et en introduisant des amorces et sondes de détection qui soient spécifiques des acides nucléiques convertis. Un kit permettant la mise oeuvre d'un tel procédé est décrit, ainsi qu'une utilisation dudit procédé ou du kit afin d'amplifier et de détecter spécifiquement des cibles bactériennes, eubactériennes, fongiques, pan-fongiques, virales ou de levures.

L'essor de la biologie moléculaire a rendu possible la manipulation des acides nucléiques. Les méthodes d'amplification génique in vitro sont alors devenues, un outil indispensable, en diagnostic biologique par exemple, en permettant de copier en grand nombre une séquence d'ADN ou d'ARN connue, avec un facteur de multiplication de l'ordre du milliard, dans un temps relativement court. Un inconvénient majeur de ces techniques réside dans l'amplification d'acides nucléiques indésirables, entraînant des résultats erronés, c'est-à-dire des résultats positifs même en l'absence de cible recherchée. C'est ce que l'on appelle des faux positifs dus à la contamination.

L'amplification de cibles bactériennes par la PCR (Polymerase Chain Reaction), la NASBA (Nucleic Acid Sequence Based Amplification), la TMA (transcription Mediated Amplification) ou n'importe quelle autre technique d'amplification de matériel génétique est une technique si sensible qu'elle nécessite l'utilisation de solutions aqueuses, enzymes, réactifs, etc., ainsi que de contenants plastiques, qui soient exempts de toutes traces d'acides nucléiques contaminants. En effet, de par la sensibilité de ces techniques, ces acides nucléiques contaminants peuvent être amplifiés et générer des faux-positifs abaissant considérablement la fiabilité du test de diagnostic. Ceci est vrai dans le cas d'amplifications bactériennes ou fongiques et est encore plus marqué dans les amplifications pan-bactériennes (également appelées eubactériennes) et pan-fongique où les amorces (et sondes) utilisées sont capables d'amplifier (et de détecter) la très grande majorité des cibles bactériennes ou fongiques. De plus dans ce cas précis, la plupart des réactifs qui rentrent dans la fabrication des kits d'amplification sont issus de sources naturelles (nucléotides, enzymes, etc.) et par conséquent, le risque potentiel de contamination par des acides nucléiques exogènes est élevé. Ainsi, dans un test, destiné à évaluer le niveau de contamination bactérienne dans un échantillon biologique et qui est réalisé par amplification, on va systématiquement générer un résultat positif conduisant à un faux diagnostic car une partie des enzymes utilisées est issue de clonage bactérien et apporte des acides nucléiques qui peuvent être amplifiés.

Cette contamination peut provenir également de l'environnement et du matériel mal décontaminé, par exemple : paillasses de laboratoire, personnels, équipements et dispositifs de pipetage, de même pour les contenants plastiques.

Un certain nombre de recommandations visant à limiter ces contamination a été mis en place. Il s'agit de méthodes préventives concernant par exemple la manipulation des échantillons (techniques de stérilisation notamment) ou encore les équipements de laboratoire (zones de travail physiquement délimitées, utilisation de hottes d'aspiration, gradient de pression entre l'extérieur et l'intérieur, afin que le flux permette toujours l'évacuation dans la direction souhaitée, etc.).

Comme évoqué précédemment, une source non négligeable de contamination réside dans les matières premières, telles qu'enzymes, réactifs, contenants plastiques, proprement dites, utilisées dans les réactions d'amplification. Ainsi, Corless C.E et al. exposent les méfaits de la contamination de la Taq polymérase sur la sensibilité de la PCR en temps réel pour la détection de l'ARN 16S (J. Clin. Microbiol. ; (2000) ; Mal ; 38(5) : 1747-52).

Une première façon de remédier à la contamination des enzymes nécessaires à l'amplification, consiste en des méthodes de décontamination enzymatique.

Une méthode enzymatique, décrite par Ashkenas S. et al. (Biotechniques ; 2005 ; Jul. ; 39(1) : 69-73), consiste à décontaminer une solution contenant l'ensemble des éléments nécessaires à une amplification. Il s'agit ici d'un cocktail d'enzymes de restriction utilisé dans le cadre d'une RT-PCR. Ces enzymes dégradent l'ADN double brin présent dans le milieu réactionnel d'amplification contenant l'ARN cible à amplifier. Elles sont ensuite inactivées par la chaleur lorsque la transcription inverse se produit. Une alternative à cette méthode pour une application PCR, donc en présence d'ADN cible double brin, est également décrite mais est limitée à l'utilisation d'un seul type d'enzyme de restriction (Type IIS RE). Toutefois, l'utilisation d'enzymes de restriction présente l'inconvénient de ne fragmenter que des acides désoxyribonucléiques double brin qui, en plus, doivent présenter le site de restriction spécifique de l'enzyme utilisée. De ce fait, les éléments contaminants sous forme simple brin et/ou ne présentant peu ou pas de tels sites de restriction ne sont alors pas éliminés.

D'autres méthodes enzymatiques de décontamination consistent, elles, à éliminer les acides nucléiques indésirables d'une solution ayant la mise en contact avec les acides nucléiques cibles. La demande de brevet WO-A-99/07887 décrit l'utilisation d'une ADNase thermolabile permettant de dégrader les acides désoxyribonucléiques double brin contenus dans le milieu réactionnel avant mise en contact avec les acides nucléiques cibles. L'enzyme est ensuite inactivée par la chaleur. L'inconvénient majeur de cette technique est que l'inactivation de cette enzyme par la chaleur dans le milieu réactionnel nécessite l'utilisation de polymérases thermostables. De plus, les réactifs présents dans le milieu réactionnel peuvent être également altérés par la température.

L'art antérieur fait état également de méthodes non-enzymatiques pour le traitement des réactifs ou matières premières.

Ainsi, Mohammadi T. et al. (J. Clin. Microbiol. ; 2003 ; Oct. ; 41(10) : 4796-8) décrit une technique consistant en la filtration sur colonnes des réactifs d'extraction et éventuellement de la digestion par une enzyme de restriction, *Sau*3AI, des réactifs de la PCR avant amplification. L'inconvénient des techniques de filtration réside dans le fait que ces techniques ne peuvent pas être appliquées à des milieux complexes sans en changer la concentration ni les propriétés. De plus, cette technique ne permet pas d'éliminer les éléments contaminants présents sur des contenants plastiques.

La demande de brevet WO-A-94/12515 décrit une méthode de traitement par un composé photoréactif d'une solution contenant la *Taq* polymérase et potentiellement des acides nucléiques contaminants. Ce composé photoréactif, par exemple un dérivé furocoumarine, est activé par une exposition aux ultra-violets. Un inconvénient majeur de cette technique, au-delà de sa mise en oeuvre contraignante, est d'être peu efficace du fait de 1a dégradation aléatoire desdits acides nucléiques et peut générer des fragments encore amplifiables. Un autre inconvénient de cette technique est qu'elle décontamine uniquement des préparations enzymatiques de la *Taq polymérase* utilisée. Les autres réactifs nécessaires à un procédé d'amplification nucléique tels que l'eau, les tampons, les contenants plastiques... doivent être décontaminés par un autre procédé technique. Ceci induit des manipulations supplémentaires consommatrices de temps et qui peuvent être onéreuses.

Une autre méthode non-enzymatique décrite par Delehanty J.B. et al., (RNA. ; 2005 ; May; 11(5) : 831-6) met en oeuvre un complexe de cobalt pour l'inhibition de la traduction. Ce complexe permet l'hydrolyse des liaisons phosphates diesters de l'ADN et de l'ARN.

Les inconvénients majeurs de cette technique sont la dégradation incomplète des acides nucléiques et la lenteur de la réaction de décontamination (24 heures).

La demande de brevet WO-A-2008/132412, de la Demanderesse, décrit un procédé permettant d'inactiver des séquences de nucléotides par l'intermédiaire de chélates métalliques. Un complexe de fragmentation, tel que le bis (1,10 phénanthroline)/Cu, est utilisé pour décontaminer les solutions, après l'étape d'amplification, de tous les acides nucléiques présents. Ceci permet d'éviter la contamination de nouveaux échantillons par des amplicons issus d'une réaction d'amplification antérieure.

Dans le cas de la première utilisation, un inconvénient majeur de cette méthode est qu'elle ne permet pas réellement la dégradation de tous les acides nucléiques contaminants, mais seulement des amplicons issus d'une réaction d'amplification antérieure. Un autre procédé devra être utilisé en amont et en parallèle à cette méthode pour décontaminer les matières premières nécessaires pour la réaction d'amplification d'acides nucléiques.

Il existe donc toujours un besoin de disposer d'une technique simple et performante de traitement d'une solution biologique ou échantillon biologique liquide, pour des acides nucléiques d'intérêt, afin que l'impact de la présence de contaminants dans l'ensemble des réactifs, ainsi que sur le matériel nécessaire à la réalisation d'une amplification nucléique, soit largement diminué voir annihilé. Ceci permet également de s'affranchir de la décontamination classique des réactifs et de l'environnement, qui sont très complexes et souvent inefficaces, car il existe un risque de re-contamination.

Les inventeurs proposent donc un tout nouveau procédé de traitement d'une solution contenant des acides nucléiques d'intérêt, qui permet, non pas la décontamination directe des réactifs, d'amplification, dont les matières premières, de la réaction d'amplification, mais de rendre l'amplification, absolument spécifique des acides nucléiques cibles d'intérêt présente dans l'échantillon biologique de départ.

Pour ce faire, la Demanderesse propose donc d'utiliser un réactif chimique ou enzymatique permettant de modifier la séquences des acides nucléiques cibles de l'échantillon biologique afin de convertir les acides nucléiques d'intérêt. Cette opération peut être réalisée juste avant l'amplification mais avant que les réactifs nécessaires à l'amplification ne soient ajoutés pour permettre ladite amplification. Les étapes d'amplification et de détection sont alors réalisées à l'aide d'amorces et de sondes de détection adaptées pour s'hybrider spécifiquement à la cible convertie mais pas aux éventuels éléments contaminants provenant par exemple des réactifs, ou de l'eau ou du contenant plastique, etc. Cette méthode permet de s'affranchir de la décontamination de l'ensemble des réactifs et du matériel utilisé depuis l'extraction des cibles jusqu'à leur amplification.

La présente invention concerne, selon un premier mode de réalisation, un procédé d'amplification permettant de s'affranchir des contaminants dans un échantillon biologique liquide contenant des acides nucléiques d'intérêt que l'on souhaite amplifier, le procédé comprenant les étapes qui suivent :
a) traiter enzymatiquement par une adénosine désaminase l'échantillon biologique pour permettre la conversion d'un type de base desdits acides nucléiques d'intérêt en un autre type de base ;
b) ajouter des amorces d'amplification, destinées à amplifier spécifiquement lesdits acides nucléiques d'intérêt convertis, chaque amorce étant constituée de trois types de bases différentes ;
c) ajouter à l'échantillon biologique, après ces traitements, les réactifs nécessaires à l'ampli.fication, tels que solution(s) aqueuse(s), solvant(s), nucléotides, enzyme(s), mais également lesdites amorces préalablement synthétisées ;
d) mettre ladite solution et les réactifs dans des conditions permettant l'amplification des acides nucléiques convertis.

La présente invention concerne également, selon un deuxième mode de réalisation, un procédé de détection permettant de s'affranchir des contaminants dans un échantillon biologique liquide contenant des acides nucléiques d'intérêt que l'on souhaite amplifier et détecter, le procédé comprenant les étapes suivantes :
a) traiter enzymatiquement par une adénosine désaminase l'échantillon biologique pour permettre la conversion d'au moins un type de base desdits acides nucléiques d'intérêt en un autre type de base ;
b) ajouter des amorces d'amplification et sonde(s) de détection, destinées respectivement à amplifier et à détecter les amplicons issus de l'amplification des acides nucléiques d'intérêt chaque amorce et sonde étant constituée de trois types de bases différentes ;
c) ajouter à l'échantillon biologique les réactifs nécessaires à l'amplification et la détection, tels que solution(s) aqueuse(s), solvant(s), nucléotides, enzyme(s), mais également lesdites amorces et sonde(s) préalablement synthétisées ;
d) mettre ladite solution et les réactifs dans des conditions permettant l'amplification des acides nucléiques convertis et la détection des amplicons générés.

Quel que soit le procédé précédent utilisé, et selon un mode préférentiel de réalisation de l'invention, on effectue au moins une étape de purification entre les étapes a) et b).

Quel que soit le procédé précédent utilisé, et toujours selon un mode préférentiel de réalisation de l'invention, préalablement à l'étape a), on effectue au moins une étape d'extraction des acides nucléiques contenus dans l'échantillon biologique liquide.

Selon n'importe lequel des cas des figures précédents, les amorces d'amplification sont spécifiques des acides nucléiques d'intérêt convertis ou de lieurs complémentaires.

Selon le deuxième mode de réalisation du procédé, selon l'invention, la ou les sondes de détection sont spécifiques de la cible convertie et des amplicons.

Dans tous les cas de figures précédents et selon un mode particulier de réalisation, les amorces ou/et les sondes constituées de trois types de bases différentes contiennent au moins un nucléotide modifié.

Selon le dernier mode de réalisation, le nucléotide modifié est choisi parmi le groupe des *alpha*-oligonucléotides, des PNA, des LNA, des 2'-O-alkyl ribonucléotides.

Selon ce dernier mode de réalisation, le nucléotide modifié est un 2'-O-méthyl ribonucléotide.

Un agent enzymatique peut être utilisé et permettre la conversion d'un type de base en un autre type de base, cet agent enzymatique est préférentiellement une adénosine désaminase.

Selon ce dernier et nouveau mode de réalisation, le traitement enzymatique consiste à convertir les adénines (A) en hypoxanthine.

Dans le cas d'un agent enzymatique, la première amorce, s'hybridant avec l'acide nucléique d'intérêt converti, est formée d'adénine(s) (A), de cytosine(s) (C) et/ou guanines (G), et que la seconde amorce, s'hybridant avec le brin résultant de l'élongation de ladite première amorce, est formée de cytosines (s) (C), de guanine(s) (G) et/ou de thymines (T).

Quel que soit le procédé utilisé, précédemment présenté, et selon un premier mode de réalisation, l'amplification réalisée est une amplification RT-PCR.

Quel que soit le procédé utilisé, précédemment présenté, et selon un second mode de réalisation, l'amplification réalisée est une amplification PCR sur un simple brin.

Quel que soit le procédé utilisé, précédemment présenté, et selon un troisième mode de réalisation, l'amplification réalisée est une amplification PCR sur un double brin.

Dans ce dernier cas, l'amplification s'effectue au moyen de deux paires d'amorces d'amplification spécifiques de chaque brin d'acide nucléique d'intérêt convertis.

Quel que soit le procédé utilisé, précédemment présenté, et selon un quatrième mode de réalisation, l'amplification réalisée est une amplification post-transcriptionnelle, telle que la NASBA ou la TMA.

Dans tous les cas de figure évoqués précédemment, les acides nucléiques d'intérêt sont des acides désoxyribonucléiques (ADN) et/ou des acides ribonucléiques (ARN).

Un kit permettant la mise oeuvre d'un procédé tel que décrit ci-dessus est également divulgué, caractérisé par le fait qu'il comprend :
a. un agent pour permettre la conversion d'au moins un type de base desdits acides nucléiques d'intérêt en un autre type de base ;
b. des amorces d'amplification, et éventuellement des sonde(s) de détection, adaptées aux acides nucléiques d'intérêt convertis ou des amplicons générés, ces séquences étant constituées de trois types de bases différents ;
c. des réactifs nécessaires à l'amplification, et éventuellement à la détection, tels que solution(s) aqueuse(s), solvant(s), nucléotides, enzyme(s).

Dans le cas d'un agent de conversion enzymatique, cet agent de conversion est l'adénosine désaminase.

La présente invention concerne enfin une utilisation du procédé, tel que décrit ci-dessus, afin d'amplifier et de détecter des cibles eubactériennes et/ou fongiques et/ou virale et/ou de levure.

Selon une variante d'utilisation, les amorces d'amplification sont spécifiques du genre bactérien et que chaque sonde de détection est spécifique d'au moins une espèce bactérienne.

Les termes suivants peuvent être indifféremment utilisés au singulier ou au pluriel.

Les termes « réactif », « réactif d'amplification », « réactif d'extraction » ou « réactif de purification » ou « matière première » désignent les réactifs, tels que les tampons de réaction, les enzymes, les nucléosides monophosphates, les solvants, les sels nécessaires à la réalisation d'une réaction d'extraction, de purification ou d'amplification enzymatique d'un acide nucléique.

Par « contenant » ou « contenant plastique » au sens de la présente invention, on entend désigner tout récipient tel que les tubes, les cônes ou embouts de pipettes, qu'ils soient en plastique (par exemple de type Eppendorf) ou en verre ou en tous autres matériaux...

Par « acide nucléique » au sens de la présence invention, on entend un enchaînement d'au moins deux nucléotides, préférentiellement au moins dix nucléotides choisis parmi les quatre types de nucléotides du code génétique, à savoir :
- le dAMP (désoxyadénosine 5'-monophosphate),
- le dGMP (désoxyguanosine 5'-monophosphate),
- le dTMP (désoxythymidine 5'-monophosphate), et
- le dCMP (désoxycytidine 5'-monophosphate),
si l'acide nucléique est un ADN, ou parmi :
- l'AMP (adénosine 5'-monophosphate),
- le GMP (guanosine 5'-monophosphate),
- l'UMP (uridine 5'-monophosphate), et
- le CMP (cytidine 5'-monophosphate),
si l'acide nucléique est un ARN.

L'acide nucléique peut également comprendre éventuellement au moins une inosine et/ou au moins un nucléotide modifié. Le terme « nucléotide modifié » signifie dans la présence invention, un nucléotide par exemple au moins un nucléotide comportant une base nucléique modifiée, la désoxyuridine, la diaimino-2,6-purine, la bromo-5-désoxyuridine ou toute autre base modifiée, préférentiellement à l'exception de la 5-méthyl-cytosine. L'acide nucléique peut aussi être modifié au niveau de la liaison internucléotidique comme par exemple les phosphorothioates, les H-phosphonates, les alkyl-phosphonates, au niveau du squelette comme par exemple les *alpha-*oligonucléotides (FR-A-2.607.507) ou les acides nucléiques polyamides (PMA) (Egholm M. et al. ; J. Am. Chem. Soc. ; 1992 ; 114 ; 1895-97) ou les 2'-O-alkyl-ribonucléotides et/ou un nucléotide 2'-O-fluoro et/ou un nucléotide 2'-amine et/ou un nucléotide arabinose, et les LNA (Sun B.W. et al., Biochemistry ; 2004 ; Apr 13 ; 43 (14) : 4160-69). Parmi les 2'-O-alkyl-ribonucléotides, les 2'-O-méthyl-ribonucléotides sont les préférés, mais on peut également utiliser les 5-Propinyl Pyrimidine Oligonucléotides (Seitz O., Angewandte Chemie International Edition 1999 ; 38(23) ; Dec : 3466-69) .

Le terme « nucléotide » définit soit un ribonucléotide soit un désoxyribonucléotide.

Au sens de la présente invention, on entend par « échantillon biologique » ou « échantillon biologique liquide », tout échantillon susceptible de contenir des acides nucléiques. Ces derniers peuvent être extraits de tissus, de sang, de sérum, de salive, de cellules circulantes d'un patient ou provenir d'un produit alimentaire, agroalimentaire ou encore être d'origine environnementale. L'extraction se fait par tout protocole connu de l'homme du métier, par exemple selon le procédé d'isolation décrit dans le brevet EP-B-0.369.063.

Par « contaminant » ou « acide contaminant » ou « acide nucléique contaminant » ou « élément contaminant », on entend au sens de la présente invention, tout acide nucléique dont l'amplification n'est pas désirée et qui est susceptible de générer un résultat faux-positif lors de la détection.

Le terme « bisulfite » signifie tout réactif chimique dont l'espèce réactive est l'ion bisulfite. L'homme du métier pourra par exemple utilisé comme réactif chimique le bisulfite de sodium (HSO₃Na), le bisulfite d'ammonium (HSO₃NH₄), le bisulfite de magnésium (HSO₃Mg), le métabisulfite de sodium (Na₂S₂O₅), l'hydrogénosulfite de sodium ou un acide sulfinique. De manière préférée, le réactif chimique est le métabisulfite de sodium.

Par « amplification » ou « réaction d'amplification » on entend toute technique d'amplification d'acides nucléiques bien connue par l'homme du métier, telle que :
- La PCR (Polymerase Chain Reaction), décrite dans les brevets US-A-4,683,195, US-A-4,683,202 et US-A-4, 800, 159, et sa dérivée RT-PCR (Reverse Transcription PCR), notamment dans un format en une étape, tel que décrit dans le brevet EP-B-0.569.272. De préférence, la PCR est réalisée sur un seul brin avec un seul couple d'amorce.
- La LCR (Ligase Chain Reaction), exposée par exemple dans la demande de brevet EP-B-0.201.184,
- La RCR (Repair Chain Reaction), décrite dans la demande de brevet WO-A-90/01069,
- La 3SR (Self Sustained Sequence Replication) avec la demande de brevet WO-A-90/06995,
- La NASBA (Nucleic Acid Sequence-Based Amplification) avec la demande de brevet WO-A-91/02818,
- La TMA (Transcription Mediated Amplification) avec le brevet US-5,399,491, et
- La RCA (Rolling Circle Amplification) décrite dans le brevet US-6,576,448.
- La RT-PCR (Reverse Transcription Polymerase Chain Reaction.

Au sens de la présente invention, on entend par « cible » ou « acide nucléique cible » ou « cible nucléique » ou « cible d'intérêt » ou « acide nucléique d'intérêt », un acide nucléique (un oligonucléotide, un polynucléotide, un fragment d'acide nucléique, un ARN ribosomique, un ARN messager, un ARN de transfert) à amplifier et/ou à détecter. La cible peut être extraite d'une cellule ou synthétisée par voie chimique. La cible peut être libre en solution ou bien liée à un support solide.

Le terme « solution » signifie une solution aqueuse homogène ou hétérogène.

Par « support solide », on entend des particules qui peuvent être en latex, en verre (CPG), en silice, en polystyrène, en agarose, en sépharose, en nylon, etc. Ces matériaux peuvent éventuellement permettre le confinememt de matière magnétique. Il peut s'agir également d'un filtre, d'un film, d'une membrane ou d'une bandelette. Ces matériaux sont bien connus de l'homme du métier.

La cible peut être un acide nucléique viral, bactérien, fongique, ou de levure, présent dans un mélange, sous forme de simple ou double brin d'ADN et/ou d'ARN. En général, la cible est d'une longueur comprise entre 50 et 10000 nucléotides, mais le plus souvent elle est comprise entre 100 et 1000 nucléotides.

Le terme « cible naturelle », dite CNT, désigne au sens de la présente invention, un acide nucléique cible à amplifier composé d'un enchaînement nucléotidique d'au moins quatre nucléotides dont les bases sont de quatre types différents et choisis parmi le groupe : adénine, guanine, cytosine et thymine (pour l'ADN) ou uracile (pour l'ARN). Eventuellement des nucléotides modifiés, tels que décrits précédemment peuvent être présents. Bien entendu les amorces dites naturelles permettant l'amplification de CNT sont appelées PNT1 et PNT2. S'il y a plusieurs amplifications en parallèle, les amorces s'appelleront PNT1a et PNT2a pour la première paire et PNT1b et PNT2b pour la seconde paire, par exemple.

Le terme « cible convertie » ou « cible quatre bases », dite C4B signifie que la cible ou l'acide nucléique cible a été traité(e) par un agent chimique ou enzymatique permettant la conversion d'un type de base portée par un nucléotide en un autre type de base différente. Le nombre et l'ordre séquentiel des nucléotides ne sont pas changés par l'action de l'agent. L'acide nucléique cible converti (C4B) a donc le même nombre de nucléotides total que l'acide nucléique cible non converti (CNT) mais est constitué d'un enchaînement de nucléotides dans lequel au moins un type de base a été modifié en un autre type de base. De manière préférée, la cible convertie sera constituée d'un enchaînement nucléotidique de type de bases choisies parmi le groupe d'adénine, thymine, guanine, uracile, cytosine et hypoxanthine.

La cible quatre bases peut donc être un acide nucléique cible à amplifier qui a été converti par le bisulfite, c'est-à-dire que soit :
- l'adénine, la guanine et la thymine sont inchangées mais la cytosine est convertie en uracile (pour l'ADN).
- l'adénine, la guanine et l'uracile sont inchangées mais la cytosine est convertie également en uracile (pour l'ARN). Dans ce cas, la conversion va permettre d'obtenir une cible convertie à trois bases, les uraciles naturellement présentes restant inchangées et les cytosines étant converties en uraciles. Cet ARN sera constitué d'un enchaînement de nucléotides constitué de trois types de bases uniquement, c'est-à-dire adénine, guanine et uracile.
Dans un souci de simplification des nomenclatures utilisées, le terme C4B sera utilisé pour les cibles ADN converties (quatre bases), mais également pour les cibles ARN converties (trois bases). Quoiqu'il en soit ceci n'a pas d'impact sur l'amplification de l'acide nucléique cible à partir de C4B, à l'aide d'amorces spécifiques dites P3B1 pour les amorces amont et dites P3B2 pour les amorces aval, l'amplicon C3B étant toujours à trois bases (voir plus bas). Là encore, dans le cas d'une amplification multiplex, les amorces spécifiques seront appelées P3B1a et P3B1b pour les amorces amont, et appelées P3B2a et P3B2b pour les amorces aval.

Cette conversion peut être le fait de la cytosine désaminase, auquel cas :
- l'adénine, la guanine et la thymine sont inchangées mais la cytosine est convertie en uracile (pour l'ADN).
- l'adénine, la guanine et l'uracile sont inchangées mais la cytosine est également convertie en uracile (pour l'ARN) (voir à ce sujet le brevet EP-B-1.654.388). Dans ce cas, la conversion va permettre d'obtenir une cible convertie à trois bases, les uraciles naturellement présentes restant inchangées et les cytosines étant converties en uraciles. Cet ARN sera constitué d'un enchaînement de nucléotides constitué de trois types de bases uniquement, c'est-à-dire adénine, guanine et uracile. Là encore, dans un souci de simplification des nomenclatures utilisées, le terme C4B sera utilisé également pour les cibles ARN converties. Quoiqu'il en soit ceci n'a pas d'impact sur l'amplification de l'acide nucléique cible à partir de C4B, à l'aide d'amorces spécifiques dites P3B1 pour les amorces amont et dites P3B2 pour les amorces aval, l'amplicon C3B étant toujours à trois bases (voir plus bas).

Cette conversion peut être le fait de l'adénosine désaminase, auquel cas :
- la cytosine, la guanine et la thymine sont inchangées mais l'adénine est transformée en hypoxanthine (pour l'ADN).
- la cytosine, la guanine et l'uracile sont inchangées mais l'adénine est transformée en hypoxanthine (pour l'ARN).
On peut citer à ce propos le document Gerber A. P. et al. Science ; 1999 ; Nov 5 ; 286 (5442) : 1146-49.

Le terme « cible trois bases », dite C3B, désigne au sens de la présente invention, un acide nucléique cible amplifié à partir de C4B, comme référence, et à l'aide d'amorces spécifiques dites P3B1 pour les amorces amont (ou « forward »), et dites P3B2 pour les amorces aval (ou « reverse »), définies plus bas.

Le terme « type de base » défini la nature de la base, c'est-à-dire soit l'adénine (A), soit la thymine (T), soit la cytosine (C), soit la guanine (G), soit l'uracile (U), soit l'hypoxanthine, non obstant le fait qu'elles soient associées au ribose (éventuellement substitué, par exemple par la présence d'un groupement 2'-O-méthyl) et éventuellement à un, deux ou trois groupements phosphates.

Par « amorce trois bases», dite P3B1 et P3B2, on entend au sens de la présente invention une séquence nucléotidique simple brin constituée d'un enchaînement d'au moins trois nucléotides, modifiés ou non, constitués de trois types de bases différentes choisies parmi le groupe d'adénine, thymine, guanine, cytosine. L'amorce trois bases sens (P3B1) est complémentaire d'au moins une partie de la séquence de l'acide nucléique cible convertie (C4B) ou d'au moins une partie de la séquence nucléotidique de l'acide nucléique synthétisée à partir de l'amorce anti-sens (amplicon) et sert de point d'initiation de la synthèse d'un acide nucléique en présence de réactifs d'amplification. L'amorce trois bases anti-sens (P3B2) est complémentaire d'au moins une partie de la séquence nucléotidique de l'acide nucléique synthétisée à partir de l'amorce sens. Ces amorces sont d'une taille comprise entre 10 et 100 nucléotides, préférentiellement entre 12 et 50 et encore plus préférentiellement entre 15 et 30 nucléotides. Selon la technique d'amplification mise en oeuvre, l'amorce pourra comprendre, en plus de la séquence d'hybridation à la cible convertie, la séquence nucléotidique d'un promoteur (par exemple T3, T7 ou SP6) dans le cas d'amplification post-transcriptionnelle, de type NASBA ou TMA. Il est bien connu de l'homme du métier que dans le cas de ces amplifications post-transcriptionnelles, l'amorce sera constituée d'une partie d'une séquence dont l'enchaînement nucléotidique sera composé de nucléotides de quatre types de bases différentes (séquence du promoteur) et d'une séquence dont l'enchaînement nucléotidique sera composé de nucléotides de trois types de bases différentes (séquence permettant l'hybridation de l'amorce sur la cible convertie).

Par « sonde de détection » ou « sonde », dite SNT, on entend une séquence nucléique d'un enchaînement nucléotidique de quatre bases de types différents choisis parmi le groupe d'adénine, thymine, guanine, uracile, cytosine, qui est capable de s'hybrider spécifiquement sur un amplicon et comportant au moins un marqueur. La sonde peut être une sonde de forme arrondie (appelée O-probe, voir demande de brevet de la Demanderesse FR08/54549 déposée le 4 juillet 2008), une balise moléculaire (autrement appelée Molecular Beacon), une sonde Taqman® ou une sonde FRET. Ces trois derniers types de sondes étant bien connus de l'homme du métier. Ces sondes peuvent éventuellement être constituées en tout ou partie de nucléotides modifiés. Chaque sonde comporte un marqueur et éventuellement un quencher.

Par « marqueur », on entend une molécule portée par un nucléotide. Le lien entre le marqueur et le nucléotide peut se faire de différentes façons connues de l'homme du métier. Le couplage manuel se fait par l'utilisation de marqueurs portant un groupement activé, typiquement un carboxyle ou un thiol, qui sont couplées sur un nucléotide interne modifié portant le groupement réactif correspondant (amine ou thiol, par exemple), ou sur une extrémité du brin nucléotidique modifiée avec ces mêmes groupements réactifs. Le couplage automatique se fait par l'utilisation de phosphoramidites portant le marqueur, et alors le couplage se fait pendant la synthèse automatisée du brin nucléotidique, soit sur une extrémité du brin, soit sur une position interne, en fonction du type de phosphoramidite utilisé. Le marqueur peut être un fluorophore ou un extincteur de fluorescence.

Par « fluorophore », on entend une molécule qui émet un signal de fluorescence quand elle est excitée par de la lumière à une longueur d'onde qui convient. Le fluorophore peut être notamment une rhodamine ou un dérivé tel que Texas Red, une fluorescéine ou un dérivé (par exemple le FAM), un fluorophore de la famille des Alexa tels que l'Alexa532 et l'Alexa647, Alexa 405, Alexa 700, Alexa 680, Cy5 ou tout autre fluorophore qui convienne en fonction de l'appareil de mesure utilisé. Les fluorophores disponibles pour les sondes de détection sont très variés et connus de l'homme du métier.

Au sens de la présente invention, on entend par « fluorescéine » une molécule chimique aromatique qui émet un signal de fluorescence avec un maximum d'émission autour de 530 nm, quand elle est excitée par de la lumière à une longueur d'onde aux alentours de 490 à 500 nm, préférentiellement de 495 nm.

Par « extincteur de fluorescence » ou « quencher », on entend une molécule qui interfère avec la fluorescence émise par un fluorophore. Cet extincteur peut être choisi parmi des molécules aromatiques non fluorescentes, pour éviter des émissions parasites. Préférentiellement, le dit extincteur est un Dabsyl ou un Dabcyl ou un « Black hole quencher™ » (BHQ) qui sont des molécules aromatiques non fluorescentes qui empêchent l'émission de fluorescence quand elles se trouvent physiquement à proximité d'un fluorophore. La technique de transfert d'énergie de fluorescence par résonance (FRET) peut également être utilisée tel que décrit par exemple dans Fluorescent Energy Transfer Nucleic Acid Probes, p. 4, Ed. V.V. Didenko, Humana Press 2006, ISSN 1064-3745. Le quencher peut également être choisi parmi des molécules fluorescentes, telles que par exemple la TAMRA (carboxytetramethylrhodamine).

La « sonde de détection trois bases » ou « sonde trois bases », dite S3B, est une sonde telle que définie précédemment et qui en plus des précédentes caractéristiques est constituée d'un enchaînement nucléotidique de trois types de bases différentes choisies parmi le groupe d'adénine, thymine, guanine, cytosine. Il est bien compris par l'homme du métier que suivant la formes des sondes (Beacon, O-probe...), la sonde sera constituée d'une partie d'une séquence dont l'enchaînement nucléotidique sera composé de nucléotides de quatre types de bases différentes et d'une séquence dont l'enchaînement nucléotidique sera composé de nucléotides de trois types de bases différentes (séquence permettant l'hybridation et la détection des amplicons).

Dans certains cas, pour améliorer l'hybridation avec les amplicons et donc leur détection, les sondes selon l'invention peuvent ponctuellement contenir de l'uracile à la place de la thymine. Dans ce cas, les sondes selon l'invention seront constituées d'un enchaînement nucléotidique de quatre types de bases différentes (uracile, guanine, adénine, thymine). Dans un souci de simplification des nomenclatures utilisées, le terme « sondes trois bases » ou S3B sera utilisé également pour ce type de sondes pour lesquelles une meilleure hybridation est recherchée. Quoiqu'il en soit ceci n'a pas d'impact sur la détection des amplicons C3B et/ou du brin complémentaire, C3Bc, les amplicons C3B et C3Bc étant toujours à trois bases (voir plus haut).

Pour mieux comprendre le principe décrit ci-dessus, nous prendrons comme exemple la cible dont la séquence hypothétique est la suivante (SEQ ID No. 1) qui suit, qui correspond à CNT. Dans le cadre d'une conversion au bisulfite, les cibles 4 bases, cibles 3 bases et amorces et sondes auront les séquences suivantes :
- CNT : 5'-AT**C**GAAATTT**CCC**GGGATCG-3', SEQ ID No. 1
- C4B : 5'-AT**U**GAAATTT**UUU**GGGAT**U**G-3', SEQ ID No. 2
- P3B1 : 3'-T**AA**C-5', SEQ ID No. 3
- C3B : 3'-TA**A**CTTTAAA**AAA**CCCTA**A**C-5', SEQ ID No. 4
- P3B2 : 5'-ATTG-3' , SEQ ID No. 5 et
- S3B : 3'-AAA**AAA**-5' , SEQ ID No. 6

La C3B a donc comme complémentaire la C3Bc :
- C3B : 3'-TA**A**CTTTAAA**AAA**CCCTA**A**C-5', SEQ ID No. 4
- C3Bc : 5'-ATTGAAATTTTTTGGGATTG-3', SEQ ID No. 7 qui est complètement différente par rapport à la CNT :
- CNT : 5'-AT**C**GAAATTT**CCC**GGGAT**C**G-3', SEQ ID No. 1
- C3Bc : 5'-AT**T**GAAATTT**TTT**GGGAT**T**G-3', SEQ ID No. 7

Par « hybridation », on entend le processus au cours duquel, dans des conditions appropriées, deux fragments nucléotidiques simple brin, ayant en tout ou partie des séquences complémentaires, sont susceptibles de former un double brin ou « duplex » stabilisé par des liaisons hydrogènes entre les bases nucléiques. Les conditions d'hybridation sont déterminées par la stringence, c'est-à-dire la rigueur et la faible salinité des conditions opératoires. L'hybridation est d'autant plus spécifique qu'elle est effectuée à plus forte stringence. La astringence est définie notamment en fonction de la composition en bases d'un duplex sonde/cible, ainsi que par le degré de mésappariement entre deux acides nucléiques. La stringence peut également être fonction des paramètres de la réaction, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépendra principalement des sondes d'hybridation utilisées. Toutes ces données sont bien connues et les conditions appropriées peuvent être déterminées par l'homme du métier.

Le terme « eqC » définit un équivalent cellule, une unité employée dans le cadre d'une amplification PCR eubactérienne et qui correspond à 1000 copies d'un ADN. Une cellule peut contenir environ 10³ copies d'ARN 16s (cible des amorces et sondes eubactériennes).

Les exemples et figures ci-joints représentent des modes particuliers de réalisation et ne peuvent pas être considérés comme limitant la portée de la présente divulgation.

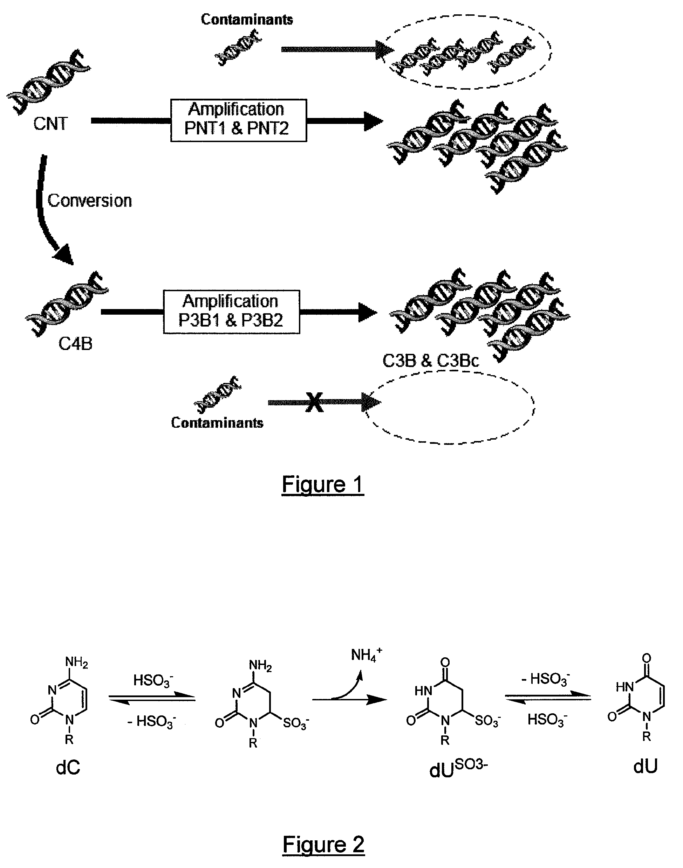

- La Figure 1 représente l'intérêt de procéder à une étape de conversion d'une cible nucléique par le bisulfite de sodium avant son amplification. Si l'amplification est réalisée de façon classique, c'est-à-dire les cibles étant naturelles (non converties dites naturelles ou CNT sur la figure) et utilisant des amorces nucléotidiques normales (dites PNT1 et PNT2 sur la figure), les éléments contaminants naturellement présents dans le mélange d'amplification sont alors co-amplifiés éventuellement avec la cible d'intérêt. En revanche, si la cible est convertie avant amplification, dite C4B, sur la figure, et que l'amplification est réalisée en utilisant des amorces nucléotidiques trois bases, dites P3B1 et P3B2, conçues pour la cible convertie, seule cette cible spécifique sera amplifiée et détectée. Bien que toujours présents dans la solution, les contaminants ne peuvent pas être amplifiés. En effet, les amorces trois bases P3B, ne peuvent pas s'hybrider sur les acides nucléiques contaminants qui comportent des nucléotides de quatre types de bases différentes, et ce même de manière non spécifique. La réaction d'amplification permet d'obtenir des amplicons dits C3B sur la figure, à partir de la cible convertie C4B, puis C3B c pour le brin complémentaire à C3B. Cette étape de conversion d'une cible nucléique par le bisulfite de sodium a un intérêt tout particulier pour les amplifications (NASBA, PCR, RT-PCR, TMA...) de cibles bactériennes, eubactériennes, fongiques, pan-fongiques, virales ou de levures.
- La Figure 2 décrit la réaction chimique qui permet la conversion de la base cytosine en base uracile (d'après Hayatsu H. ; Mut. Research ; 2008 ; 659 : 77-82).
- La Figure 3 représente les spectres d'analyse de la composition d'un ADN par spectrométrie de masse par ionisation electrospray (ESI). L'analyse a été effectuée sur une cible ADN avant (a) et après (b) la conversion par le bisulfite de sodium. L'axe des abscisses représente le temps en minute et l'axe des ordonnées représente l'absorbance en unité arbitraire. La valeur M+H représente la masse molaire d'une molécule à laquelle est ajoutée la masse d'une mole de proton. Par exemple, la présence de désoxycytosine est démontrée par l'apparition d'une masse correspondant à cette molécule.

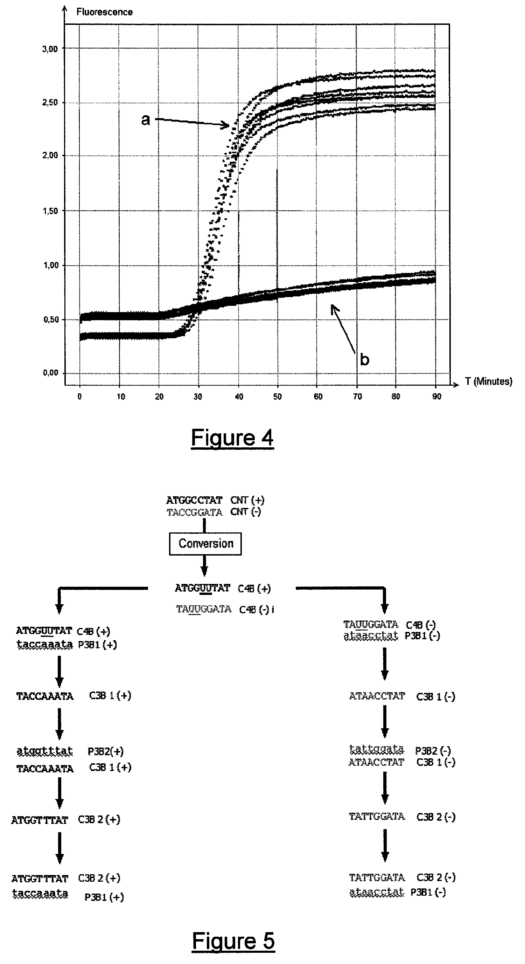

- La Figure 4 est une comparaison de témoins négatifs en amplification NASBA eubactérienne (la cible est remplacée par de l'eau) réalisée avec différents types d'amorces d'amplification (PNT ou P3B définies précédemment). La détection des amplicons s'effectue en temps réel par la mesure de la fluorescence à 488 nm en unité arbitraire (RFU pour Related Fluorescence Unit) sur l'axe des ordonnées ; l'axe des abscisses représentant le temps qui s'écoule en minutes. Les conditions expérimentales (a) correspondent à une amplification et une détection du témoin négatif avec des amorces (PNT) et une sonde de détection naturelle (SNT). Les conditions expérimentales (b) correspondent à une amplification et une détection du témoin négatif avec des amorces trois bases (P3B) et une sonde de détection trois bases (S3B).
- La Figure 5 représente schématiquement une amplification par PCR lorsque la cible convertie est un ADN double-brin. Deux couples d'amorces P3B sont nécessaires à la réalisation de cette amplification.
- La Figure 6 montre une comparaison de gammes de dilution de cibles en amplification NASBA eubactérienne. La détection des amplicons s'effectue en temps réel par la mesure de la fluorescence à 488 nm (en unité arbitraire RFU, axe des ordonnées) en fonction du temps (minutes, axe des abscisses) :
   - Figure 6A : Amplification (amorces PNT) et détection (sondes SNT) d'une gamme de dilution s'étend de 0 à 10^{e}7 copies d'une cible synthétique composée de quatre types de bases différentes (CNT).
   - Figure 6B : Amplification (amorces P3B) et détection (sondes S3B) d'une gamme de dilution s'étend de 0 à 10^{e}7 copies d'une cible synthétique composée de trois types de bases différentes (C3Bc).
- La Figure 7 représente une amplification PCR eubactérienne. La détection des amplicons s'effectue à chaque fin de cycle par la mesure de la fluorescence à 488 nm (en unité arbitraire RFU). L'axe des abscisse représente le nombre de cycle et l'axe des ordonnées représente la fluorescence en unité arbitraire, avec :
   - Figure 7A : Amplification (amorces PNT) et détection (sondes SNT) d'une gamme de dilution s'étend de 0 à 10^{e}5 copies d'une cible synthétique composée de quatre types de bases différentes (CNT).
   - Figure 7B : Amplification (amorces P3B) et détection (sondes S3B) d'une gamme de dilution s'étend de 0 à 10^{e}5 copies d'une cible synthétique composée de trois types bases différentes (C3Bc).
- La Figure 8 est une amplification PCR eubactérienne d'une gamme de dilution de l'ADNg d'*Escherichia coli.* La détection des amplicons s'effectue à chaque fin de cycle par la mesure de la fluorescence à 488 nm, comme déjà évoqué ci-dessus. L'axe des abscisse représente le nombre de cycle et l'axe des ordonnées représente la fluorescence en unité arbitraire comme précédemment décrit :
   - Figure 8A : Amplification (amorces PNT) et détection (sondes SNT) d'une gamme de dilution s'étend de 0 à 10^{e}5 copies de l'ADNg d'*Escherichia coli* (CNT).
   - Figure 8B : Amplification (amorces P3B) et détection (sondes S3B) d'une gamme de dilution s'étend de 0 à 10^{e}5 copies de l'ADNg d'*Escherichia coli* converti par un traitement au bisulfite (C4B).

En relation avec les figures 1 et 2, et plus précisément, la conversion de la cible par le bisulfite de sodium est réalisée de la façon suivante. Il s'agit d'un protocole composé de quatre étapes :
(1) Tout d'abord, la conversion débute par une dénaturation de La cible à la soude (NaOH) puis ajout du bisulfite de sodium et de l'hydroquinone (celle-ci permet de limiter l'oxydation du bisulfite).
(2) La cible est ensuite purifiée par filtration sur colonne.
(3) La cible est désulfonée en milieu basique.
(4) Enfin une dernière purification sur colonne permet d'obtenir une cible convertie prête à être amplifiée.

Il est déjà connu dans l'état de la technique de réaliser chimiquement la conversion d'acides nucléiques à l'aide de bisulfite. A ce propos, on trouve :
1) Des travaux très anciens de Shapiro et de Hayatsu avec entre autres :
   - « Reaction of Cytosine and Uracil with sodium bisulfite » Shapiro R. et al, J. Biol. Chem. ; 1973 ; Jun ; 248 : 4060-64, et
   - « The addition of sodium bisulfite to uracil and to cytosine » Hayatsu H. et al, J. Am. Chem. Soc. ; 1970 ; 40(26) : 724-26.
   Ceci prouve que de nombreux documents ont été publiés dans ce domaine mais sans vraiment cerner, à cette époque, les implications qu'il y aurait dans le diagnostic.
2) Des demandes de brevets concernant la technique de traitement de l'ADN au bisulfite pour utiliser un groupe d'amorces non dégénérées afin d'amplifier un ensemble d'acides nucléiques appartenant à une même espèce d'origine :
   - bactérienne (WO-A-2006/058393 et WO-A-2007/140506), et
   - virale (WO-A-2007/030882).
3) Une amélioration du procédé de traitement de l'acide nucléique au bisulfite pour la détection des motifs de méthylation des séquences génomiques de l'ADN « High Sensitivity mapping of methylated cytosines » de Clark S. J. , Ncleic. Acids Res. ; 1994 August 11 ; vol 22 (15) : 2990-97.
4) Un nouveau développement du procédé de traitement d'un acide nucléique par le bisulfite, le traitement étant adapté à des acides nucléiques fixés sur des supports solides (EP-B-1.590.362 et EP-A-1.394.173).
5) Une publication qui résume l'état de la technique du traitement d'un acide nucléique par le bisulfite pour le séquençage de l'ADN génomique et l'identification de motifs de cytosines méthylées. Cette synthèse a été écrite par le développeur de la technologie bisulfite : Hayatsu H., Mut. Research ; 2008 ; 659 : 77-82.

Il est donc clair que la chimie de conversion au bisulfite est déjà bien décrite et ce depuis plus de trente cinq ans maintenant. Par contre la conversion chimique ou enzymatique utilisée afin de s'affranchir des acides nucléiques contaminants dus aux réactifs utilisés lors d'une extraction, d'une purification et d'une amplification d'un acide nucléique cible n'est absolument pas décrite ou même évoquée.

Notre divulgation concerne donc des amplifications spécifiques de la cible convertie sans bruit de fond dû aux acides nucléiques contaminants.

L'objectif est donc de disposer d'un procédé possédant les avantages suivants :
1) simple et rapide,
2) utilisable juste avant l'étape d'amplification,
3) capable de convertir la cible avec un rendement élevé,
4) être compatible avec de très nombreuses techniques d'amplification dont la PCR, ainsi que les amplifications dites post-transcriptionnelles (NASBA et TMA).
5) être automatisable et adapté à un support solide et/ou magnétique.

Les exemples 1-4 suivants sont des exemples de référence.

### Exemple 1 : Démonstration de la conversion d'une cible nucléique par le bisulfite :

### Objectif :

Montrer la conversion par le bisulfite d'une cible biologique ayant les quatre types de bases A, T, G, C, soit A (pour Adénine), T (pour Thymine), G (pour Guanine) et C (pour Cytosine) en cible à quatre bases A, T, G et U (pour Uracile).

### Mode opératoire :

La conversion est réalisée avec le kit commercial ZYMO - EZ DNA Methylation-GoldTM Kit, ZYMO Research, #D5005 (Orange, CA 92867 - Etats Unis d'Amérique), en suivant le protocole fourni avec le kit.

La cible à convertir est un échantillon d'ADN génomique commercial extrait de la bactérie *Escherichia coli* 0157, #IRMM449-1EA, Sigma-Aldrich Chimie (L'Isle d'Abeau Chesnes - FRANCE) de 500 à 4000 nucléotides double brin.

Conversion par le bisulfite : On suit le protocole du kit selon lequel, 130 µl de réactif CT Conversion Reagent présent sont ajoutés à 20 µl d'échantillon (ce qui correspond à 200 ng d'ADN génomique extrait d'*Escherichia coli*). L'ensemble est incubé 10 minutes à 98°C, afin de permettre la dénaturation des cibles du génome d'*Escherichia coli,* puis 150 minutes à 64°C et 5 minutes à 4°C (Thermocycler Applied Biosystems GeneAmp 9700, Foster city, U.S.A.). Ce qui donne une solution contenant une cible convertie.

Purification : 600 µl de tampon de fixation appelé Binding buffer dans le kit, sont ajoutés aux 150 pi de solution contenant la cible convertie. Le mélange est déposé sur la colonne fournie dans le kit et centrifugé 30 secondes à 10000xg. Un volume de 100 µl de tampon de lavage, appelé Wash buffer, est déposé sur la colonne qui est centrifugée 30 secondes à 12000xg.

Désulfonation : Un volume de 200 µl de tampon de désulfonation, dit Desulfonation buffer, est déposé sur la même colonne qui est incubée 15 minutes à température ambiante puis centrifugée 30 secondes à 12000xg. La colonne est ensuite lavée deux fois par ajout de 200 µl de tampon de lavage ou Wash buffer et est centrifugée durant 30 secondes à 12000xg. Elution : La colonne est déposée sur un tube propre. Un volume de 10 µl de tampon d'élution, appelé M-Elution buffer, est déposé au centre de la colonne qui est centrifugée 30 secondes à 12000xg. Les 10 µl d'éluat contiennent l'ADN converti par le bisulfite, prêt à l'emploi pour une amplification.

Les échantillons d'ADNg d'*Escherichia coli* convertis sont ensuite hydrolysés par un mélange de Nucléase P1 (13 U) (N8630-1VL, Sigma Aldrich, St Louis, U.S.A.) et de Phosphatase Alcaline (3 U) (P7923-2KU, Sigma Aldrich, St Louis, U.S.A.) pendant 1 nuit à 37°C. L'ADN génomique hydrolysé est ensuite analysé par HPLC.

### Conditions HPLC et de détection par spectrométrie de masse à ionisation electrospray.

Pour ce faire, on utilise :
- une chaîne HPLC WATERS Alliance 2795 (Milford, Connecticut (CT) USA),
- une colonne WATERS XTerra MS C18 (Milford, CT, USA) 4,6 x 30 2,5 µm, utilisée avec un débit de 1 ml/minute à 30°C (détection à 260 nm) avec un gradient linéaire d'acétonitrile de : 0% à 5% (4 min); 5% à 12% (5 min); 12% à 90% (2 min) et 90% à 0% (3 min) dans 10 mM de formiate d'ammonium à pH 7.
- un détecteur à barrette de diodes PDA 996, logiciel Empower version 2 (Milford, CT, USA),
- un détecteur de masse (ZQ Electrospray WATERS ((Milford, CT, USA).

### Conclusion :

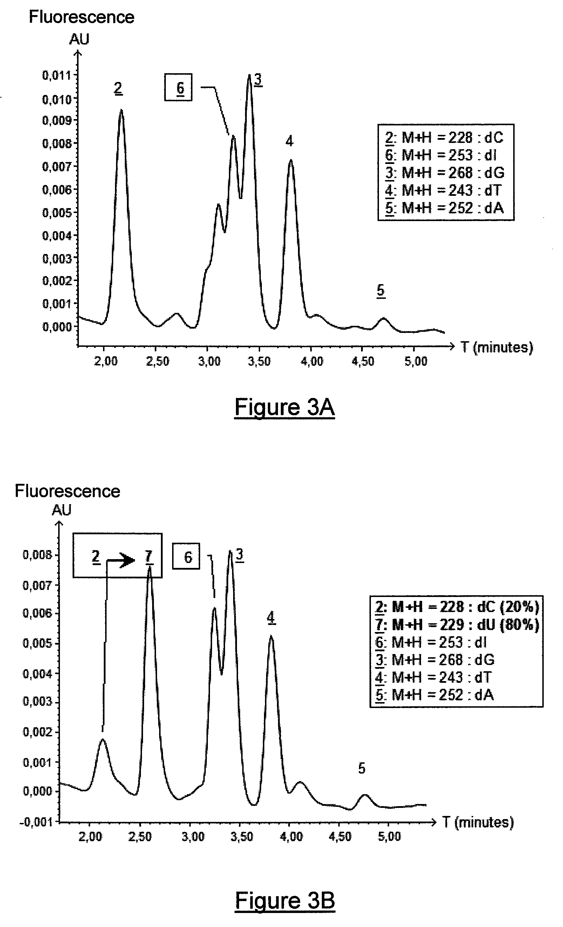

La Figure 3(a) montre l'analyse de la composition d'un ADNg hydrolysé d'*Escherichia coli* non converti par le bisulfite par spectrométrie de masse par ionisation electrospray (ESI). Il est possible d'observer quatre pics principaux correspondant aux quatre types de nucléosides : désoxyadénosine dA, désoxythimidine dT, désoxyguanosine dG, désoxycytidine dC avec une trace de désoxyinosine dI. La désoxyinosine provient de l'action enzymatique de l'Adénosine Désaminase sur la désoxyadénosine. L'Adénosine Désaminase est un contaminant que l'on trouve sous forme de trace dans les préparations commerciales de la Phosphatase Alcaline.

La Figure 3(b) montre l'analyse de la composition d'un ADNg hydrolysé d'*Escherichia coli* après conversion par le bisulfite. On constate l'apparition d'un nouveau pic d'élution (pic 7) correspondant à la désoxyuridine dU et la quasi-disparition du pic de dC (pic 2). Les autres pics d'élutions (pics 3, 4 et 5) sont inchangés et correspondent à la présence de dG, dT et dA. Ce chromatogramme montre que cet ADN est composé de quatre nucléosides qui sont : dU, dA, dT et dG.
Dans cet exemple, le rendement de conversion dans ces conditions expérimentales est de 80%. Ce rendement est fonction de la taille de l'ADN d'intérêt que l'on souhaite traiter. Le taux de conversion (ou le taux de rendement) est beaucoup plus élevé sur un fragment court d'ADN. Ici dans nos conditions expérimentales, l'ADN traité est de l'ADN génomique de *E*. *coli*, c'est à dire un ADN cible de grande longueur, assez difficile à dénaturer et à convertir. Cependant, la démonstration de la conversion de la majorité des dC en dU est donc faite.

### Exemple 2 : Réalisation d'une amplification NASBA eubactérienne avec des amorces d'amplification quatre bases (PNT) ou trois bases (P3B) à partir d'une gamme de dilution d'oligonucléotides synthétiques trois ou quatre bases (appelées respectivement C3B et CNT):

### Objectif :

Faire la preuve du concept, c'est à dire mettre en évidence qu'une NASBA eubactérienne réalisée à l'aide d'amorces trois bases (P3B1 et P3B2) et de sondes bases (S3B) permet d'amplifier et de détecter spécifiquement une cible synthétique trois bases sans amplifier les cibles naturelles (CNT), comme les cibles bactériennes contaminantes.

La détection s'effectue en temps réel à l'aide de sonde de détection quatre bases (SNT) ou de sonde de détection trois bases (S3B).

### Mode opératoire :

Cet essai est réalisé en procédant à une amplification NASBA eubactérienne avec d'une part :
- une gamme de dilution de cibles oligonucléotides quatre bases dite CNT, des amorces quatre bases (dites PNT1 et PNT2) et une sonde de détection quatre bases (SNT), les quatre types de bases étant A, T, G et C ;
et d'autre part :
- une gamme de dilution de cibles oligonucléotides trois bases dite C3B, des amorces (P3B1 et P3B2) et une sonde de détection 3 bases (S3B) pouvant s'hybrider avec C3B.

Les gammes de dilutions vont de 0 à 10⁷ copies de cibles par essai.

Les cibles oligonucléotidiques, les amorces et les sondes ont été commandées tels que, sans conversion au bisulfite, chez Eurogentec, Seraing, Belgique) et ont pour séquence :
Cible CNT (SEQ ID No. 8) :
Amorces PNT1 (SEQ ID No. 9) :
   5'-aattctaatacgactcactataggGCGGGACTTAACCCAACATC-3'
Amorces PNT2 (SEQ ID No. 10) :
   5'-GGAGCATGTGGTTTAATTCG-3'
Sonde de détection SNT (SEQ ID No. 11) :
   5'-**FAM-cgatcg**TWTCGTCAGCTCGTGT**cgatcg-Dabcyl**-3' avec W = 2'-O-Me-guanosine.
cible C3B (SEQ ID No. 12) :
Amorce P3B1 (SEQ ID No. 13) :
   5'-aattctaatacgactcactataggACAAAACTTAACCCAACATC-3'
Amorce P3B2 (SEQ ID No. 14) :
   5'-GGAGTATGTGGTTTAATTTG-3'
Sonde de détection S3B (SEQ ID No. 15) :
   5 '**-FAM-cgatcg**TWTTGTTAGTTTGTGT**cgatcg-Dabsyl**-3' avec W = 2'-O-Me-guanosine.

Dans ces séquences, la séquence du promoteur T7 correspond aux lettres en minuscule ; la sonde de détection étant un Molecular Beacon, la séquence de la boucle est présentée en lettre majuscule et les séquences des tiges sont présentées en lettre minuscule et en gras.

L'amplification NASBA est réalisée en suivant les instructions du fabriquant fournies dans le kit NASBA NucliSENS EasyQ VIH-1 vl. 2(Réf. 285 036, bioMérieux, Marcy l'Etoile, France). Briévement, les mélanges d'amplification (Mix) et le mélange d'enzymes sont préparés de la manière suivante :

Mélange d'amplification (quantité pour huit tubes) :
- eau de qualité NASBA, dit NASBA water : 11,2 µl,
- diluant des réactifs dit Reagent diluent : 64 µl,
- : 12,8 µl,
- sphère de réactifs : 1 sphère, et
- mélange d'amorces et de sonde : 8 µl.

Mélange d'enzymes (quantité pour huit tubes) :
- diluant des enzymes dit Enzyme diluent : 45 µl (pour 8 tubes), et
- sphère d'enzymes : 1 sphère.

Assemblage : Un volume de 1.0 µl de mélange d'amplification est déposé dans un tube de 0,2 ml auquel est ajouté un volume de 5 µl de cible nucléiques. Un volume de 5 µl de mélange d'enzymes est déposé dans le bouchon du tube. Le tube est fermé et incubé 5 minutes à 65°C puis 5 minutes à 41°C. Le tube est brièvement centrifugé, sans agitation, pour réunir les deux mélanges puis incubé dans un fluorimètre NucliSens EasyQ (Réf. 200309, bioMérieux, Marcy l'Etoile, France) pendant 90 minutes à 41°C (programme standard QL1-90). La lecture de la fluorescence s'effectue à 488 nm pendant la réaction.

On constate que l'amplification NASBA de la cible CNT, correspondant à la Figure 4, courbes (a), amplifie très fortement les acides nucléiques contaminants alors que le signal reste très faible sur la Figure 4, courbes (b), dans la cas d'une amplification NASBA de la cible quatre bases. Ce qui prouve que les contaminants ne sont pas ou peu amplifiés par les amorces P3B1 et P3B2, et qu'ils ne sont pas ou peu détectés par la sonde S3B.

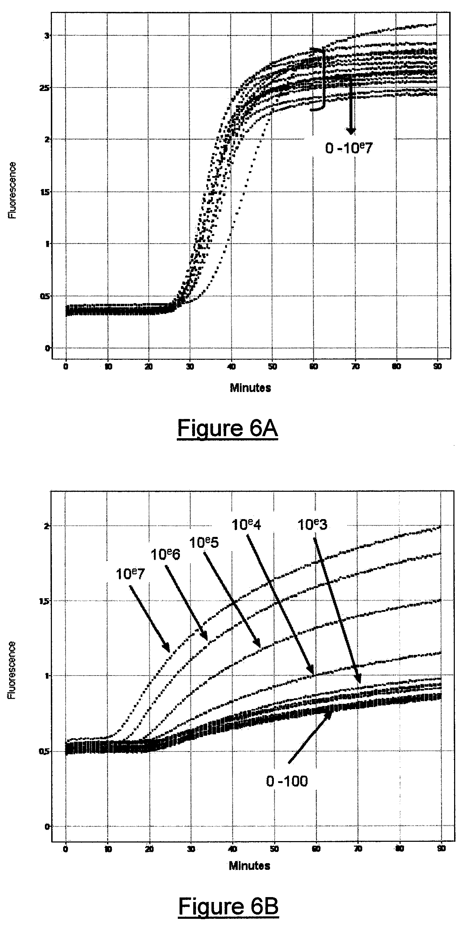

La Figure 6A montre que l'amplification NASBA réalisée avec les amorces PNT1 et PNT2 ne permet pas de différencier la cible spécifique CNT (à des concentrations de 0 à 10⁷ copies par essai) du témoin négatif (0 copie par essai).

A l'inverse, l'amplification NASBA réalisée avec les amorces P3B1. et P3B2 sur une cible trois bases C3B (Figure 6B) permet d'obtenir une gamme de dilution de la cible spécifique C3B avec une bonne sensibilité de détection. Dans cet exemple, la sensibilité est de 10³ copies par essai.

### Conclusion :

Cette expérience montre clairement l'intérêt que représente cette amplification sur cibles trois bases, car elle permet un gain de sensibilité en détection de cibles d'environ quatre log. Dans l'expérience de l'amplification NASBA de la cible CNT, le témoin négatif est identifié avec un signal correspondant à environ 10^{e}7 copies/essai alors que ce signal est inférieur à 10^{e}3 copies/essai dans l'amplification NASBA de la cible C3B.

### Exemple 3 : Réalisation d'une amplification PCR eubactérienne avec des amorces PNT ou P3B à partir d'une gamme de dilution de cibles oligonucléotidiques synthétiques trois ou quatre bases :

### Objectif :

Mettre en évidence le gain de sensibilité de détection d'une cible bactérienne synthétique trois bases (C3B) après amplification par une PCR eubactérienne utilisant des amorces trois bases (P3B) spécifiques des cibles trois bases (C3B) par rapport à une amplification par PCR eubactérienne sur une cible naturelle (CNT) avec des amorces quatre bases (PNT) et une sonde quatre bases (SNT).

### Mode opératoire:

L'amplification PCR, eubactérienne est réalisée à l'aide d'amorces et sondes de détection fluorescente dite Taqman®, SNT ou S3B. Les cibles sont des oligonucléotides synthétiques de quatre-vingt-douze nucléotides possédant une séquence quatre bases (A, T, G, C pour CNT ; SEQ ID No. 8) ou trois bases (A, T, G pour C3B ; SEQ ID No. 12). Les amplifications sont réalisées sur des dilutions de cibles variant de 0 à 10⁵ copies par essai.

L'amplification est réalisée selon les instructions du fabriquant fournie dans le kit Roche LightCycler FastStart DNA Master Hyprobe, #030003248001 (Bâle, Suisse). Les séquences des amorces et sonde utilisées sont les suivantes :

### PCR sur une cible naturelle, cible CNT :

- Amorce PNT1a (SEQ ID No. 16) :
   5'-AGGATAAGGGTTGCGCTCGTTGCGGG-3'
- Amorce PNT2a (SEQ ID No. 17) :
   5'-TGGAGCATGTGGTTTAATTC-3'
- Sonde TaqMan® SNTa (SEQ ID No. 18) :
   5'-FAM-TWTCGTCAGCTCGTGT-BHQ1-3' avec W = 2'-OMe-G

### PCR sur une cible trois bases, cible C3B :

- Amorce P3B1a (SEQ ID No. 19%) :
   5'-AAAATAAAAATTACACTCATTACAAA-3'
- Amorce P3B2a (SEQ ID No. 20) :
   5'-TGGAGTATGTGGTTTAATTT-3'
- Sonde TaqMan® S3Ba (SEQ ID No. 21) :
   5'-FAM-TGTTGYYKWYYYGTGT-BHQ1-3' avec Y = 2'-OMe-U, W = 2'-OMe-G et K = 2' -OMe-A.

Les nucléotides 2'-O-Me permettent de compenser la perte de la température d'hybridation liée à la disparition des bases C dans les séquences des sondes trois bases. L'introduction ponctuelle d'uracile (en l'espèce, uracile modifiée) dans le design de cette sonde TaqMan® permet d'améliorer l'hybridation avec les amplicons.

### Mélange d'amplification : (pour un tube)

- Eau PCR : 10,4 µl,
- Amorce P1 (10 µM) : 1 µl,
- Amorce P2 (10 µM) : 1 µl,
- Sonde (2,5 µM) : 2 µl,
- MgCl₂ (25 mM) : 1,6 µl, et
- Mélange d'amplification (10x Master Mix) : 2 µl.

Le mélange d'amplification final est pré-incubé à 95°C pendant 10 minutes, puis amplifier sur 45 cycles composés d'étapes de dénaturation à 95°C pendant 10 secondes, d'hybridation à 50°C pendant 15 secondes et d'élongation à 60°C pendant 15 secondes. L'amplification est stoppée par une incubation de 5 minutes à 95°C. La lecture de la fluorescence s'effectue à 530 nm, pendant le cycle d'élongation.

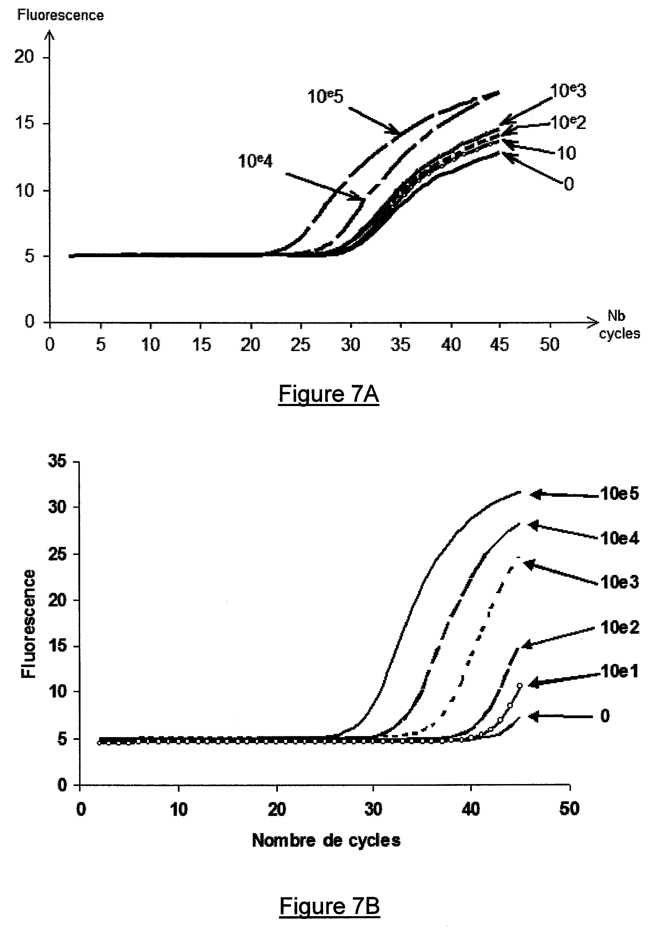

La Figure 7A indique qu'en dessous de 10³ copies de cibles CNT par essai, le signal PCR est indifférenciable du signal du témoin négatif (0 copies) . La sensibilité d'une PCR eubactérienne sur une cible CNT est donc de 10³ copies par essai.

Sur la Figure 7B, l'amplification PCR eubactérienne sur cibles oligonucléotidiques trois bases (C3B) montre une excellente sensibilité avec une courbe qui reste horizontale pour le témoin négatif (0 copie par essai) et une sensibilité supérieure à 10 copies par essai.

### Conclusion :

Cette expérience démontre que le recours à l'amplification PCR eubactérienne sur cibles synthétiques trois bases C3B permet un gain de sensibilité de quatre log.

La sensibilité de l'amplification d'une cible trois bases est indépendante de là technique d'amplification et des moyens de détection utilisés. Ces expériences montrent que quelque soit la technique d'amplification et la forme des sondes de détection trois bases employées, la sensibilité d'une amplification d'une cible trois bases selon la présente divulgation est très supérieure à celle d'une amplification classique d'une cible naturelle (CNT).

### Exemple 4 : Démonstration du gain de sensibilité apporté par la conversion de la cible ADN bactérienne et amplification PCR à l'aide d'amorces P3B :

### Objectif :

Après démonstration sur modèle synthétique (exemples 2 et 3), un essai est réalisé sur un vrai modèle biologique converti expérimentalement par le bisulfite. L'objectif est de mettre en évidence le gain de sensibilité de détection d'une cible bactérienne convertie puis amplifiée par une PCR utilisant des amorces d'amplification P3B spécifiques des cibles converties.

### Mode opératoire :

Cette démonstration est réalisée en utilisant un kit de conversion commercial Zymo Research et selon les instructions du fournisseurs du kit (cf. exemple 1). La conversion est réalisées sur de l'extrait d'ADN génomique d*'Escherichia coli* (O157, Sigma IBMM449-1EA) composé d'acides nucléiques de 50 à 4000 nucléotides. Les amplifications PCR eubactériennes sont réalisés dans les mêmes conditions expérimentales que celles décrites dans l'exemple 3, avec les mêmes amorces et les mêmes sondes de détection. La lecture de la fluorescence s'effectue à 530 nm pendant le cycle de l'élongation.

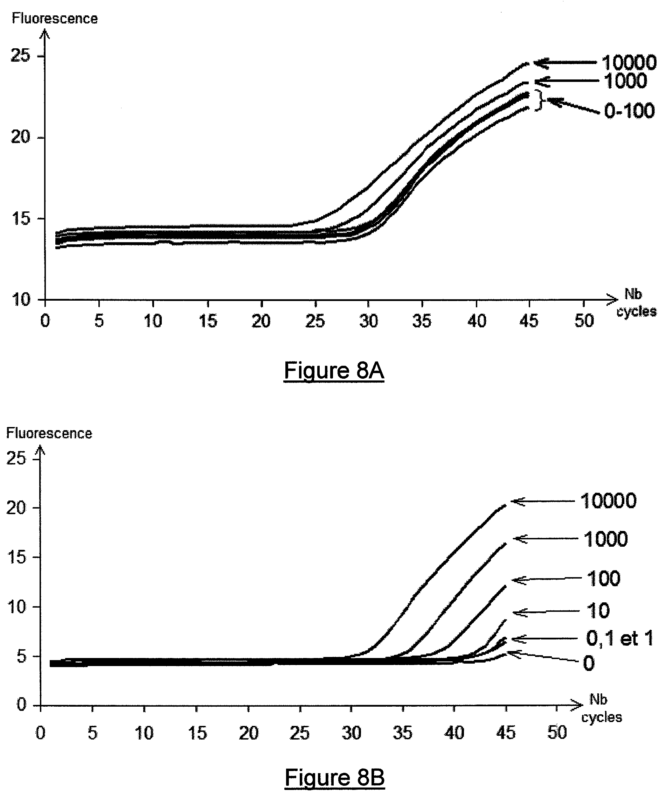

La figure 8A correspond aux conditions expérimentales dans lesquelles l'ADN cible de *E*. *coli* n'a pas été traité au bisulfite. Cet ADN est donc constitué d'un enchaînement de nucléotides de quatre types de bases différentes, à savoir A, T, G et C. L'amplification de cet ADN génère des amplicons constitués d'un enchaînement de quatre types de bases différentes (A, T, G et C).

La figure 8B correspond aux conditions expérimentales dans lesquelles l'ADN de *E*. *coli* a été converti après un traitement au bisulfite. Cet ADN converti (cible convertie 4 bases) se compose d'un enchaînement de nucléotides de quatre types de bases différents, à savoir A, T, G, U. L'amplification de cet ADN converti avec les amorces précédemment décrites (amorces 3 bases) va générer des amplicons constitués d'un enchaînement nucléotides de trois types de bases différent : A, T, C ou A, T, G.

Comme indiqué dans la Figure 8A, dans le cas d'une amplification PCR eubactérienne d'un ADN d*'Escherichia coli* non converti, le niveau de sensibilité est de 1000 eqC par essai. Le témoin négatif à 0 eqC par essai est confondu avec le 100 eqC par essai. Cette figure montre qu'en dessous d'un seuil de 100 000 de copies d'un ADN cible, il n'est pas possible de distinguer l'ADN cible des ADN contaminants présents dans les différents réactifs utilisés.

La Figure 8B indique qu'après conversion de l'échantillon par le bisulfite et amplification PCR eubactérienne, le niveau de sensibilité obtenu est de 0,1 à 1 eqC par essai. Le témoin négatif donne un signal très faible. Cette expérience démontre que le procédé selon l'invention permet de s'affranchir de tous éléments contaminants et d'obtenir ainsi un gain de sensibilité très significatif.

De plus, bien que le rendement de conversion de l'ADN génomique de *E*. *coli* dans nos conditions expérimentales soit de 80% (cf plus haut), la sensibilité de détection est relativement élevée et ne semble pas être affectée par les 20 % d'ADN génomique de *E. coli* qui n'ont pas été converti. Ces 20% d'ADN génomique n'ayant pas eu une modification au niveau leur types de bases, ils deviennent *ipso facto* des contaminants et ne sont pas amplifiés par le procédé tel que décrit.

### Conclusion :

Cet exemple d'amplification PCR eubactérienne sur une cible d'ADN bactérien convertie par le bisulfite démontre clairement le gain de sensibilité que peut apporter ce traitement de l'échantillon. En effet, le gain de sensibilité est de quatre log. Le recours à une amplification avec traitement préalable au bisulfite permet d'éteindre les signaux des témoins négatifs; même si le taux de rendement du traitement de conversion n'est pas proche de 100%.

Il s'agit donc d'une méthode de choix pour s'affranchir complètement des élément contaminants bactériens présents dans les réactifs d'extraction, de purification et d'amplification.

La portée de la présente invention est limitée par les revendications 1-14.

### SEQUENCE LISTING

<110> bioMérieux
<120> Bisulfite Sequences
<130> Bisulfite
<160> 21
<170> PatentIn version 3.3
<210> 1
   <211> 20
   <212> DNA
   <213> Artificial
<220>
   <223> target
<400> 1
   atcgaaattt cccgggatcg 20
<210> 2
   <211> 20
   <212> DNA
   <213> Artificial
<220>
   <223> converted target
<220>
   <221> misc_feature
   <222> (3)..(3)
   <223> n in uracil
<220>
   <221> misc_feature
   <222> (11)..(11)
   <223> n is uracil
<220>
   <221> misc_feature
   <222> (12)..(12)
   <223> n is uracil
<220>
   <221> misc_feature
   <222> (13)..(13)
   <223> n is uracil
<220>
   <221> misc_feature
   <222> (19) .. (19)
   <223> n is uracil
<400> 2
   atngaaattt nnngggatng 20
<210> 3
   <211> 4
   <212> DNA
   <213> Artificial
<220>
   <223> primer
<400> 3
   caat 4
<210> 4
   <211> 20
   <212> DNA
   <213> Artificial
<220>
   <223> amplicon
<400> 4
   caatcccaaa aaatttcaat 20
<210> 5
   <211> 4
   <212> DNA
   <213> Artificial
<220>
   <223> primer
<400> 5
   attg 4
<210> 6
   <211> 6
   <212> DNA
   <213> Artificial
<220>
   <223> probe
<400> 6
   aaaaaa 6
<210> 7
   <211> 20
   <212> DNA
   <213> Artificial
<220>
   <223> amplicon
<400> 7
   attgaaattt tttgggattg 20
<210> 8
   <211> 92
   <212> DNA
   <213> Artificial
<220>
   <223> target
<400> 8
   tggagcatgt ggtttaattc gctacaactg tcgtcagctc gtgttccgcg ggatgttggg 60
   ttaagtcccg caacgagcgc aacccttatc ct 92
<210> 9
   <211> 44
   <212> DNA
   <213> Artificial
<220>
   <223> promoter + primer
<400> 9
   aattctaata cgactcacta tagggcggga cttaacccaa catc 44
<210> 10
   <211> 20
   <212> DNA
   <213> Artificial
<220>
   <223> primer
<400> 10
   ggagcatgtg gtttaattcg 20
<210> 11
   <211> 28
   <212> DNA
   <213> Artificial
<220>
   <223> probe
<220>
   <221> misc_feature
   <222> (8)..(8)
   <223> w = 2'-O-methyl-guanosin.
<400> 11
   cgatcgtwtc gtcagctcgt gtcgatcg 28
<210> 12
   <211> 92
   <212> DNA
   <213> Artificial
<220>
   <223> target
<400> 12
   tggagtatgt ggtttaattt gttataattg ttgttagttt gtgttttgtg ggatgttggg 60
   ttaagttttg taatgagtgt aatttttatt tt 92
<210> 13
   <211> 44
   <212> DNA
   <213> Artificial
<220>
   <223> promoter + primer
<400> 13
   aattctaata cgactcacta taggacaaaa cttaacccaa catc 44
<210> 14
   <211> 20
   <212> DNA
   <213> Artificial
<220>
   <223> primer
<400> 14
   ggagtatgtg gtttaatttg 20
<210> 15
   <211> 28
   <212> DNA
   <213> Artificial
<220>
   <223> probe
<220>
   <221> misc_feature
   <222> (8)..(8)
   <223> w = 2'-O-methyl-guanosin.
<400> 15
   cgatcgtwtt gttagtttgt gtcgatcg 28
<210> 16
   <211> 26
   <212> DNA
   <213> Artificial
<220>
   <223> primer
<400> 16
   aggataaggg ttgcgctcgt tgcggg 26
<210> 17
   <211> 20
   <212> DNA
   <213> Artificial
<220>
   <223> primer
<400> 17
   tggagcatgt ggtttaattc 20
<210> 18
   <211> 16
   <212> DNA
   <213> Artificial
<220>
   <223> probe
<220>
   <221> misc_feature
   <222> (2)..(2)
   <223> w = 2'-O-methyl-guanosin.
<400> 18
   twtcgtcagc tcgtgt 16
<210> 19
   <211> 26
   <212> DNA
   <213> Artificial
<220>
   <223> primer
<400> 19
   aaaataaaaa ttacactcat tacaaa 26
<210> 20
   <211> 20
   <212> DNA
   <213> Artificial
<220>
   <223> primer
<400> 20
   tggagtatgt ggtttaattt 20
<210> 21
   <211> 16
   <212> DNA
   <213> Artificial
<220>
   <223> probe
<220>
   <221> misc_feature
   <222> (6)..(6)
   <223> y = 2'-O-methyl-uracil
<220>
   <221> misc_feature
   <222> (7)..(7)
   <223> y = 2'-O-methyl-uracil
<220>
   <221> misc_feature
   <222> (8)..(8)
   <223> k = 2'-O-methyl-adenin
<220>
   <221> misc_feature
   <222> (9)..(9)
   <223> w = 2'-O-methyl-guanosin
<220>
   <221> misc_feature
   <222> (10)..(10)
   <223> y = 2'-O-methyl-uracil
<220>
   <221> misc_feature
   <222> (11)..(11)
   <223> y = 2'-O-methyl-uracil
<220>
   <221> misc_feature
   <222> (12)..(12)
   <223> y = 2'-O-methyl-uracil
<400> 21
   tgttgyykwy yygtgt 16

## Revendications

1. Procédé d'amplification spécifique des acides nucléiques d'intérêt de cibles bactériennes, eubactériennes, fongiques, pan-fongiques, virales ou de levures présents dans un échantillon biologique liquide, procédé comprenant les étapes qui suivent :
a) traiter enzymatiquement par une adénosine désaminase l'échantillon biologique pour permettre la conversion d'un type de base desdits acides nucléiques d'intérêt en un autre type de base ;
b) ajouter des amorces d'amplification, destinées à amplifier spécifiquement lesdits acides nucléiques d'intérêt convertis, chaque amorce étant constituée de trois types de bases différentes ;
c) ajouter à l'échantillon biologique, après ces traitements, les réactifs nécessaires à l'amplification, tels que solution(s) aqueuse(s), solvant(s), nucléotides, enzyme(s), mais également lesdites amorces préalablement synthétisées ;
d) mettre ladite solution et les réactifs dans des conditions permettant l'amplification des acides nucléiques convertis,
ledit procédé permettant, d'une part, de s'affranchir des contaminants présents dans l'ensemble des réactifs ainsi que sur le matériel nécessaire à la réalisation d'une amplification nucléique et, d'autre part, de s'affranchir de la décontamination de l'ensemble des réactifs et dudit matériel.

2. Procédé de détection spécifique des acides nucléiques d'intérêt de cibles bactériennes, eubactériennes, fongiques, pan-fongiques, virales ou de levures présents dans un échantillon biologique liquide, le procédé comprenant les étapes suivantes :
a) traiter enzymatiquement par une adénosine désaminase l'échantillon biologique pour permettre la conversion d'au moins un type de base desdits acides nucléiques d'intérêt en un autre type de base ;
b) ajouter des amorces d'amplification et sonde(s) de détection, destinées respectivement à amplifier et à détecter les amplicons issus de l'amplification des acides nucléiques d'intérêt chaque amorce et sonde étant constituée de trois types de bases différentes ;
c) ajouter à l'échantillon biologique les réactifs nécessaires à l'amplification et la détection, tels que solution(s) aqueuse(s), solvant(s), nucléotides, enzyme(s), mais également lesdites amorces et sonde(s) préalablement synthétisées ;
d) mettre ladite solution et les réactifs dans des conditions permettant l'amplification des acides nucléiques convertis et la détection des amplicons générés,
ledit procédé permettant, d'une part, de s'affranchir des contaminants présents dans l'ensemble des réactifs ainsi que sur le matériel nécessaire à la réalisation d'une amplification nucléique et, d'autre part, de s'affranchir de la décontamination de l'ensemble des réactifs et dudit matériel.

3. Procédé, selon l'une quelconque des revendications 1 ou 2, **caractérisé en ce que** l'on effectue au moins une étape de purification entre les étapes a) et b).

4. Procédé, selon l'une quelconque des revendications. 1 à 3, **caractérisé en ce que**, préalablement à l'étape a), on effectue au moins une étape d'extraction des acides nucléiques contenus dans l'échantillon biologique liquide.

5. Procédé, selon l'une quelconque des revendications 1 à 4, **caractérisé en ce que** les amorces d'amplification sont spécifiques des acides nucléiques d'intérêt convertis ou de leurs complémentaires.

6. Procédé, selon la revendication 2, **caractérisé en ce que** la ou les sondes de détection sont spécifiques de la cible convertie et des amplicons.

7. Procédé, selon l'une quelconque des revendications 1 à 6, **caractérisé en ce que** les amorces ou/et les sondes constituées de trois types de bases différentes contiennent au moins un nucléotide modifié.

8. Procédé, selon l'une quelconque des revendications 1 à 7, **caractérisé en ce que** la première amorce, s'hybridant avec l'acide nucléique d'intérêt converti, est formée d'adénine (s) (A), de cytosine(s) (C) et/ou guanines (G)-et que la seconde amorce, s'hybridant avec le brin résultant de l'élongation de ladite première amorce, est formée de cytosines (s) (C), de guanine (s) (G) et/ou de thymines (T).

9. Procédé, selon l'une quelconque des revendications 1 à 8, **caractérisé en ce que** l'amplification réalisée est une amplification RT-PCR.

10. Procédé, selon l'une quelconque des revendications 1 à 8, **caractérisé en ce que** l'amplification réalisée est une amplification PCR sur un simple brin.

11. Procédé, selon l'uné quelconque des revendications 1 à 8, **caractérisé en ce que** l'amplification réalisée est une amplification PCR sur un double brin.

12. Procédé, selon la revendication 11 **caractérisé en ce que** l'amplification s'effectue au moyen de deux paires d'amorces d'amplification spécifiques de chaque brin d'acide nucléique d'intérêt convertis.

13. Procédé, selon l'une quelconque des revendications 1 à 8, **caractérisé en ce que** l'amplification réalisée est une amplification post-transcriptionnelle, telle que la NASBA ou la TMA.

14. Procédé, selon l'une quelconque des revendications 1-8 ou 13, **caractérisé en ce que** les acides nucléiques d'intérêt sont des acides désoxyribonucléiques (ADN) et/ou des acides ribonucléiques (ARN).

## Patentansprüche

1. Verfahren zur spezifischen Amplifikation von Nukleinsäuren von Interesse von bakteriellen, eubakteriellen, fungalen, panfungalen, viralen Zielen oder von Hefen, die in einer flüssigen biologischen Probe vorhanden sind, wobei das Verfahren die folgenden Schritte umfasst:
a) enzymatische Behandlung der biologischen Probe mit einer Adenosindesaminase, um die Umwandlung eines Basentyps der Nukleinsäuren von Interesse in einen anderen Basentyp zu ermöglichen;
b) Zugabe von Amplifikationsprimern zum spezifischen Amplifizieren der umgewandelten Nukleinsäuren von Interesse, wobei jeder Primer aus drei verschiedenen Basentypen besteht;
c) Zugabe, zu der biologischen Probe nach diesen Behandlungen, der Reagenzien, die für die Amplifikation notwendig sind, wie wässrige Lösung(en), Lösungsmittel, Nukleotide, Enzym(e), aber auch die zuvor synthetisierten Primer;
d) Überführen der Lösung und der Reagenzien in Bedingungen, die die Amplifikation der umgewandelten Nukleinsäuren gestatten,
wobei es das Verfahren einerseits gestattet, Verunreinigungen loszuwerden, die in den gesamten Reagenzien sowie auf der zur Durchführung einer Nukleinsäureamplifikation benötigten Substanz vorhanden sind, und andererseits die Dekontamination der gesamten Reagenzien und der Substanz überflüssig zu machen.

2. Verfahren zum spezifischen Nachweis von Nukleinsäuren von Interesse von bakteriellen, eubakteriellen, fungalen, panfungalen, viralen Zielen oder von Hefen, die in einer flüssigen biologischen Probe vorhanden sind, wobei das Verfahren die folgenden Schritte umfasst:
a) enzymatische Behandlung der biologischen Probe mit einer Adenosindesaminase, um die Umwandlung mindestens eines Basentyps der Nukleinsäuren von Interesse in einen anderen Basentyp zu ermöglichen;
b) Zugabe von Amplifikationsprimern und einer/von Nachweissonde(n) zum Amplifizieren beziehungsweise Nachweisen der aus der Amplifikation des Nukleinsäuren von Interesse hervorgegangenen Amplikons, wobei jeder Primer und jede Sonde aus drei verschiedenen Basentypen besteht;
c) Zugabe, zu der biologischen Probe, der Reagenzien, die für die Amplifikation und den Nachweis notwendig sind, wie wässrige Lösung(en), Lösungsmittel, Nukleotide, Enzym(e), aber auch die zuvor synthetisierte(n) Primer und Sonde(n);
d) Überführen der Lösung und der Reagenzien in Bedingungen, die die Amplifikation der umgewandelten Nukleinsäuren und den Nachweis der hergestellten Amplikons gestatten,
wobei es das Verfahren einerseits gestattet, Verunreinigungen loszuwerden, die in den gesamten Reagenzien sowie auf der zur Durchführung einer Nukleinsäureamplifikation benötigten Substanz vorhanden sind, und andererseits die Dekontamination der gesamten Reagenzien und der Substanz überflüssig zu machen.

3. Verfahren nach einem der Ansprüche 1 oder 2, **dadurch gekennzeichnet, dass** man mindestens einen Reinigungsschritt zwischen den Schritten a) und b) durchführt.

4. Verfahren nach einem der Ansprüche 1 bis 3; **dadurch gekennzeichnet, dass** man vor Schritt a) mindestens einen Schritt der Extraktion der in der flüssigen biologischen. Probe enthaltenen Nukleinsäuren durchführt.

5. Verfahren nach einem der Ansprüche 1 bis 4, **dadurch gekennzeichnet, dass** die Amplifikationsprimer für die umgewandelten Nukleinsäuren von Interesse oder deren Komplemente spezifisch sind.

6. Verfahren nach Anspruch 2, **dadurch gekennzeichnet, dass** die Nachweissonde(n) für das umgewandelte Ziel und die Amplikons spezifisch sind.

7. Verfahren nach einem der Ansprüche 1 bis 6, **dadurch gekennzeichnet, dass** die aus drei verschiedenen Basentypen bestehenden Primer und/oder Sonden mindestens ein modifiziertes Nukleotid enthalten.

8. Verfahren nach einem der Ansprüche 1 bis 7, **dadurch gekennzeichnet, dass** der erste Primer, der an die umgewandelte Nukleinsäure von Interesse hybridisiert, aus Adenin(en) (A), Cytosin(en) (C) und/oder Guanin(en) (G) hergestellt ist, und dass der zweite Primer, der an den Strang hybridisiert, der aus der Verlängerung des ersten Primers hervorgeht, aus Cytosin(en) (C), Guanin(en) (G) und/oder Thymin(en) (T) hergestellt ist.

9. Verfahren nach einem der Ansprüche 1 bis 8, **dadurch gekennzeichnet, dass** es sich bei der durchgeführten Amplifikation um eine RT-PCR-Amplifikation handelt.

10. Verfahren nach einem der Ansprüche 1 bis 8, **dadurch gekennzeichnet, dass** es sich bei der durchgeführten Amplifikation um eine PCR-Amplifikation an einem Einzelstrang handelt.

11. Verfahren nach einem der Ansprüche 1 bis 8, **dadurch gekennzeichnet, dass** es sich bei der durchgeführten Amplifikation um eine PCR-Amplifikation an einem Doppelstrang handelt.

12. Verfahren nach Anspruch 11, **dadurch gekennzeichnet, dass** man die Amplifikation mithilfe zweier Amplifikationsprimerpaare durchführt, die für jeden umgewandelten Nukleinsäurestrang von Interesse spezifisch sind.

13. Verfahren nach einem der Ansprüche 1 bis 8, **dadurch gekennzeichnet, dass** es sich bei der durchgeführten Amplifikation um eine posttranskriptionale Amplifikation, wie NASBA oder TMA, handelt.

14. Verfahren nach einem der Ansprüche 1-8 oder 13, **dadurch gekennzeichnet, dass** es sich bei den Nukleinsäuren von Interesse um Desoxyribonukleinsäuren (DNA) und/oder Ribonukleinsäuren (RNA) handelt.

## Claims

1. A method of amplification specific of nucleic acids of interest of bacterial, eubacterial, fungal, pan-fungal, viral or yeast targets present in a liquid biological ample, said method comprising the following stages:
a) treating the biological sample enzymatically by an adenosine deaminase to permit conversion of one type of base of said nucleic acids of interest to another type of base;
b) adding amplification primers, intended for specifically amplifying said converted nucleic acids of interest, each primer being, constituted of three different types of bases;
c) adding to the biological sample, after these treatments, the reagents necessary for the amplification, such as aqueous solution(s), solvent(s), nucleotides, enzyme(s), but also said primers previously synthesized;
d) placing said solution and the reagents in conditions permitting amplification of the converted nucleic acids,
said method allowing, on one side, to overcome the presence of contaminants in all of the reagents, as well as on the equipment required for carrying out a nucleic acid amplification and, on the other side, to make it unnecessary to decontaminate all of said reagents, as well as of said equipment.

2. A method of detection specific of nucleic acids of interest of bacterial, eubacterial, fungal, pan-fungal, viral or yeast targets in a liquid biological sample, said method comprising the following stages:
a) treating the biological sample enzymatically by an adenosine deaminase to permit conversion of at least one type of base of said nucleic acids of interest to another type of base;
b) adding amplification primers and detecting probe(s), intended respectively for amplifying and for detecting the amplicons resulting from amplification of the nucleic acids of interest, each primer and probe being constituted of three different types of bases;
c) adding, to the biological sample, the reagents required for the amplification and detection, such as aqueous solution(s), solvent(s), nucleotides, enzyme(s), but also said primers and probe(s) previously synthesized;
d) placing said solution and the reagents in conditions permitting amplification of the nucleic acids converted and detection of the amplicons generated,
said method allowing, on one side, to overcome the presence of contaminants in all of the reagents, as well as on the equipment required for carrying out a nucleic acid amplification and, on the other side, to make it unnecessary to decontaminate all of said reagents, as well as of said equipment.

3. The method as claimed in either of claims 1 and 2, **characterized in that** at least one stage of purification is carried out between stages a) and b).

4. The method as claimed in any one of claims 1 to 3, **characterized in that** at least one stage of extraction of the nucleic acids contained in the liquid biological sample is carried out prior to stage a).

5. The method as claimed in any one of claims 1 to 4, **characterized in that** the amplification primers are specific to the converted nucleic acids of interest or to those complementary to them.

6. The method as claimed in claim 2, **characterized in that** the detecting probe or detecting probes are specific to the converted target and to the amplicons.

7. The method as claimed in any one of claims 1 to 6, **characterized in that** the primers and/or probes constituted of three different types of bases contain at least one modified nucleotide.

8. The method as claimed in any one of claims 1 to 7, **characterized in that** the first primer, hybridizing to the converted nucleic acid of interest, is formed from adenine(s) (A), cytosine(s) (C) and/or thymines (T), and **in that** the second primer, hybridizing to the strand resulting from the elongation of said first primer, is formed from adenine(s) (A), guanine(s) (G) and/or thymines (T).

9. The method as claimed in any one of claims 1 to 8, **characterized in that** the amplification carried out is an RT-PCR amplification.

10. The method as claimed in any one of claims 1 to 8, **characterized in that** the amplification carried out is a PCR amplification on a single strand.

11. The method as claimed in any one of claims 1 to 8, **characterized in that** the amplification carried out is a PCR amplification on a double strand.

12. The method as claimed in claim 11, **characterized in that** amplification is effected by means of two pairs of amplification primers specific to each strand of converted nucleic acid of interest.

13. The method as claimed in any one of claims 1 to 8, **characterized in that** the amplification carried out is a post-transcriptional amplification, such as NASBA or TMA.

14. The method as claimed in any one of claims 1 to 8 or 13, **characterized in that** the nucleic acids of interest are deoxyribonucleic acids (DNA) and/or ribonucleic acids (RNA).
